# EUROPEAN PATENT APPLICATION

(11) **EP 3 295 960 A1**
(43) Date of publication of application: **21.03.2018**
(21) Application number: 16792957.9
(22) Date of filing: 10.05.2016
(51) Int. Cl.: A61K 48/00, A61K 31/713, A61K 41/00

(54) **COMPOSITION FOR CANCER CELL SENSITIZATION CONTAINING AS ACTIVE INGREDIENT SUBSTANCE INHIBITING EXPRESSION OF ONCOGENE OF HPV VIRUS**

(30) Priority: 12.05.2015 KR 20150066218
(71) Applicant: Abion Inc., Gangnam-gu, Seoul 06309 (KR)
(72) Inventor: SHIN, Young Kee, Seoul 06279 (KR); KIM, Young Deug, Incheon 22241 (KR); JUNG, Hun Soon, Seoul 07733 (KR); KIM, Deuk Ae, Seoul 08801 (KR); HA, Tae Kyung, Daegu 42006 (KR)
(74) Representative: Isarpatent
(86) International application number: PCT/KR2016/004867
(87) International publication number: WO 2016/182311

(57) **Abstract**

The present invention relates to a composition for cancer cell sensitization containing as an active ingredient a substance inhibiting the expression of an oncogene of a HPV virus, and more specifically, to a composition for radiosensitization which is used for the treatment of cancer induced by HPV infection and contains as an active ingredient oligonucleotide having any one nucleic acid sequence selected from the group consisting of sequence numbers 1 to 10. The oligonucleotide of the present invention inhibits the expressions of E6 and E7 which are HPV virus oncoproteins, thereby inhibiting the proliferation of tumor cells, and enhances the sensitivity of tumor cells to radiation, thereby exhibiting an excellent effect as a sensitizer that can maximize the cell death effect.

## Description

### [TECHNICAL FIELD]

The present invention relates to a composition for cancer cell sensitization containing as an active ingredient a substance inhibiting the expression of oncogene of HPV virus, and more particularly to a radiation-sensitive composition comprising an oligonucleotide having any one of the nucleic acid sequences selected from the group consisting of SEQ ID NOs: 1 to 10 as an active ingredient and used for the treatment of cancer induced by HPV infection.

### [BACKGROUND ART]

This application claims priority from Korean Patent Application No. 10-2015-0066218 filed on May 12, 2015, the entire contents of which are incorporated herein by reference.

High-risk human papillomavirus (HPV) types 16 and 18 are the major causes of cervical cancer and cervical dysplasia, and cause other genital cancers and head and neck squamous cell carcinoma. Cervical cancer is one of the most common types of malignant tumors in women. Although the incidence of invasive cervical cancer is declining slowly, it is the most frequent cancer in all developing countries, accounting for 25% of female cancers. HPV is a small DNA virus that causes benign malignant tumors with approximate sequences of 8,000 nucleotides. To date, more than 100 subtypes of HPV have been identified by genomic differences, and about 90 genotypes of HPV are completely analyzed. Of these types, the high-risk HPV types (eg, HPV-16,18, 31, 33, 35, 45, 51, 52, 56) are responsible for almost 90% of cervical cancer. More than 50% of cervical cancer infected with HPV is associated with HPV type 16, followed by HPV type 18 (12%), HPV type 45 (8%) and HPV type 31 (5%). These HPVs encode two oncogenic proteins, E6 and E7. All of these proteins are involved in HPV-mediated cell immortalization and cell transformation. The oncogenic E6 protein binds to the wild type of p53 tumor suppressor protein and degrades p53 through the ubiquitin pathway.

On the other hand, the E7 protein binds directly to Rb and causes hyper-phosphorylation. First, E6 forms a complex with E6-AP (E6_associated protein), which is E3-ubiquitin-protein ligase. Subsequently, the E6 / E6-AP complex binds to wild-type p53 and blocks ubiquitination of p53-mediated cell responses to DNA damage. Mainly, p53 tumor suppressor protein is regulated by Mdm2-mediated ubiquitination, but in HPV-infected cervical cancer cells, degradation of p53 is completely replaced with E6-mediated ubiquitination from Mdm2-mediated ubiquitination. Thus, unlike many other cancers, almost all of HPV-infected cervical cancer has the wild-type of p53 genes. However, the expression level of the p53 protein is very low, since it is consistently degraded by E6 proteins. In particular, the HPV E6 protein has received considerable attention because it is a specific target that can kill only cervical cancer cells. These strategies, targeting E6 or E6 / E6-AP complexes, involve several treatments.

The use of cytotoxic drugs, inhibitors that release zinc from the E6 oncogenic proteins of the virus, epitope peptides mimicking E6-AP (mimotope), anti-E6 ribozymes, peptide aptamer targeting the E6 oncogenic proteins of the virus, siRNA targeting the E6 oncogenic gene of the virus, and the combination treatment thereof have been indicated. Recently, it has been proven that siRNA selectively silences internal genes in animal cells as well as silences genes of viruses among diseases caused by viruses. RNA interference (RNAi) caused by transfection of siRNAs has emerged as a new treatment for treating human viral infections. In HPV-infected cervical cancer cells, siRNAs targeting E6 and E7 genes cause accumulation of p53 and pRb leading to apoptosis or cell senescence. It revealed that in the HPV-16-infected cervical cancer cell line and the HPV-18-infected cell line, RNAi targeting the oncogenic genes of virus E6 and E7 selectively silenced the expression of these proteins.

### [DETAILED DESCRIPTION OF THE INVENTION]

### [TECHNOLOGICAL ASSIGNMENT]

The inventors of the present invention sought to find the siRNA specific to E6 / E7, which is capable of achieving synergistic effect by being treated together during the radiotherapy, and as a result of efforts as exemplified in the specification, it has been found that siRNAs prepared by hybridizing a sense oligonucleotide selected from the group consisting of SEQ ID NOs: 1 to 10 and an antisense oligonucleotide complementarily binding to the sense sequence exhibit significantly higher synergistic effect than that of the single treatment due to the combination therapy in the case of performing radiotherapy.

Accordingly, an aspect of the present invention is to provide a sensitizing composition for treating HPV(human papilloma virus)-induced cancer with radiation therapy, comprising an oligonucleotide having any one of the nucleic acid sequences selected from the group consisting of SEQ ID NOs: 1 to 10 as an active ingredient.

Another aspect of the present invention is to provide a sensitizing agent for treating HPV(human papilloma virus)-induced cancer with radiation therapy, comprising an oligonucleotide having any one of the nucleic acid sequences selected from the group consisting of SEQ ID NOs: 1 to 10 as an active ingredient.

Another aspect of the present invention is to provide a use of an oligonucleotide having any one of the nucleotide sequences selected from the group consisting of SEQ ID NOs: 1 to 10 for preparing a sensitizing agent for treating HPV(human papilloma virus)-induced cancer with radiation therapy.

Another aspect of the present invention is to provide a sensitizing method for treating HPV(human papilloma virus)-induced cancer with radiation therapy, the method comprising administering an effective amount of a composition, which comprises an oligonucleotide having any one of the nucleic acid sequences selected from the group consisting of SEQ ID NOs: 1 to 10, to a subject in need thereof.

Another aspect of the present invention is to provide a method of for treating HPV(human papilloma virus)-induced cancer with radiation therapy, the method comprising steps (a) administering an effective amount of a composition, which comprises an oligonucleotide having any one of the nucleic acid sequences selected from the group consisting of SEQ ID NOs: 1 to 10, to a subject in need thereof; and (b) performing a radiotherapy on said subject.

### [TECHNOLOGICAL RESOLUTION]

In one embodiment, according to the present invention, the present invention provides a sensitizing composition for treating HPV(human papilloma virus)-induced cancer with radiation therapy, comprising an oligonucleotide having any one of the nucleic acid sequences selected from the group consisting of SEQ ID NOs: 1 to 10 as an active ingredient.

In another embodiment, according to the present invention, the present invention provides a sensitizing agent for treating HPV(human papilloma virus)-induced cancer with radiation therapy, comprising an oligonucleotide having any one of the nucleic acid sequences selected from the group consisting of SEQ ID NOs: 1 to 10 as an active ingredient.

In another embodiment, according to the present invention, the present invention provides a use of an oligonucleotide having any one of the nucleotide sequences selected from the group consisting of SEQ ID NOs: 1 to 10 for preparing a sensitizing agent for treating HPV(human papilloma virus)-induced cancer with radiation therapy.

In another embodiment, according to the present invention, the present invention provides a sensitizing method for treating HPV(human papilloma virus)-induced cancer with radiation therapy, the method comprising administering an effective amount of a composition, which comprises an oligonucleotide having any one of the nucleic acid sequences selected from the group consisting of SEQ ID NOs: 1 to 10, to a subject in need thereof.

In another embodiment, according to the present invention, the present invention provides a method of for treating HPV(human papilloma virus)-induced cancer with radiation therapy, the method comprising steps (a) administering an effective amount of a composition, which comprises an oligonucleotide having any one of the nucleic acid sequences selected from the group consisting of SEQ ID NOs: 1 to 10, to a subject in need thereof; and (b) performing a radiotherapy on said subject.

Hereinafter, the present invention will be described in detail.

The present invention provides a sensitizing composition for treating HPV(human papilloma virus)-induced cancer with radiation therapy, comprising an oligonucleotide having any one of the nucleic acid sequences selected from the group consisting of SEQ ID NOs: 1 to 10 as an active ingredient.

The present invention also provides a sensitizing agent for treating HPV(human papilloma virus)-induced cancer with radiation therapy, comprising an oligonucleotide having any one of the nucleic acid sequences selected from the group consisting of SEQ ID NOs: 1 to 10 as an active ingredient.

The radiation-sensitizing composition or the sensitizing agent refers to a composition capable of improving the damage sensitivity of cancer cells to radiation when treating cancer, thereby further increasing the therapeutic effect. In other words, it means a composition or an agent which can inhibit the resistance of cancer cells to radiation to facilitate the induction of the death of cancer cells by radiation.

In the present invention, the cancer caused by the HPV infection is selected from the group consisting of cervical cancer, vagina cancer, vulvar cancer, anal cancer, penis cancer, tonsil cancer, pharynx cancer, larynx cancer, head and neck cancer, and lung adenocarcinoma. Preferably, the cancer is cervical cancer, but is not limited thereto.

In the present invention, the oligonucleotide is characterized by being a siRNA (small interfering RNA) complementarily hybridized with a sense oligonucleotide and a complementary antisense oligonucleotide, but is not limited thereto. The siRNA of the present invention may have a structure in which a sense strand and an antisense strand are located on opposite sides to form a double strand. In addition, the siRNA molecules of the present invention may have a single-stranded structure with self-complementary sense and antisense strands.

Preferably, the present invention provides a composition wherein the oligonucleotide is a small interfering RNA (siRNA) complementarily hybridized with a sense oligonucleotide and a complementary antisense oligonucleotide.

The oligonucleotides of the present invention can inhibit the expression of E6 and E7, the oncogenic proteins of HPV virus. Prior to the present invention, the inventors of the present invention had developed an siRNA capable of specifically binding to two oncogenic proteins E6 and E7 of High-risk Human Papilloma Virus (HPV) types 16 and 18 (Korean Patent No. 10-1197627).

On the other hand, according to Bertrand research group, it has been found that siRNA has superior inhibitory effect on mRNA expression in vitro and in vivo compared to antisense oligonucleotide (ASO) and has longer lasting effects of the inhibition as compared to antisense oligonucleotides (ASO) when siRNA and oligonucleotide (ASO) are present on the same target gene (Comparison of antisense oligonucleotides and siRNAs in cell culture and in vivo. Biochem. Biophys. Res.Commun. 2002. 296 : 1000-1004). In addition, since the action mechanism of siRNA binds complementarily to the target mRNA to regulate the expression of the target gene in a sequence-specific manner, it has the advantage of being able to expand the scope of applications that can be applied compared to conventional antibody-based drugs or small molecule drugs (Progress Towards in vivo Use of siRNAs. MOLECULAR THERAPY. 2006 13(4) : 664-670).

Despite the excellent effects of siRNAs and their various application ranges, in order for siRNA to be developed as a therapeutic agent, the stability of siRNA in the body should be improved and siRNA should be effectively delivered to target cells through improvement of cell delivery efficiency (Harnessing in vivo siRNA delivery for drug discovery and therapeutic development. Drug Discov Today. 2006 Jan ; 11(1-2) : 67-73).

In order to solve the above problem, studies have been actively made on modification of some nucleotides or backbone of siRNA, so as to have a nucleic acid-degrading enzyme resistance or utilization of carriers, such as a viral vector, a liposome, or a nanoparticle, to improve the stability in the body.

The present invention provides oligonucleotides that have undergone vari ous modifications for imparting nucleic acid-degrading enzyme resistance and reducing nonspecific immune responses, in order to improve the in vivo stability of oligonucle otides. The modifications of oligonucleotides can be used in combination with on e or more modifications selected from modifications by substitution of the OH gr oup at the 2' carbon position of the sugar structure in one or more nucleotides with -CH3 (methyl), -OCH3 (methoxy), -NH2, -F (fluorine), -O-2-methoxyethyl-O-propyl, -O-2-methylthioethyl, -O-3-aminopropyl, -O-3-dimethylaminopropyl, -ON-methylacetamido or -O-dimethylamidooxyethylo group ; substitution of the oxygen in the sugar structure of a nucleotide with sulfur; or by change nucleotide bond into phosphorothioat e or boranolophosphate, methyl phosphonate bond, while modifications in the fo rm of a peptide nucleic acid (PNA), a locked nucleic acid (LNA), or an unlocked n ucleic acid (UNA) can also be used (Ann. Rev. Med. 55, 61-65 2004 ; US 5,660, 985; US 5,958,691; US 6,531,584; US 5,808,023; US 6,326,358; US 6,175,001 ; B ioorg. Med. Chem. Lett. 14:1139-1143, 2003 ; RNA, 9 : 1034-1048, 2003 ; Nuc leic Acid Res. 31:589- 595, 2003 ; Nucleic Acids Research, 38(17) 576175773, 2010; Nucleic Acids Research, 39(5) 182371832, 2011). Preferably, the modified base in the present invention comprises one or more modifications selected from the group consisting of methylation, glycosylation, and halogenation. Mo re preferably, the base modified in the present invention is a 2'-O-methylated or 2'-fluorinated base.

As used herein, 2'-O methylation means that the hydroxyl group bound to carbon atom 2 of the ribose in the RNA molecule is substituted by a methyl group and is transformed into a 2'-methoxy group, and 2'-fluorination means that the hydroxy group bound to the carbon atom 2 of the ribose of the RNA molecule is substituted by a fluoro group and is transformed into a 2'-fluoro group.

According to a preferred example in the present invention, the 2'-O methylated base of the nucleotide in the present invention is U or G.

According to a preferred example in the present invention, the 2'-O fluorinated base of the nucleotide in the present invention is C.

According to one example of the present invention, the present inventors produced modified siRNAs by introducing a 2'-OMe and / or 2'-F modification into the oligonucleotide disclosed in Korean Patent No. 10-1197627, which is a prior art.

According to one example of the present invention, the HeLa cells, the HPV type 18 positive cervical cancer cell line, or SiHa cells, the HPV type 16 positive cervical cancer cell line, were treated with chemically modified siRNA to evaluate inhibitory ability in tumor cell proliferation and inhibitory ability in expression of TP53, E6 and E7 protein. As a result, the siRNAs of the present invention showed excellent inhibitory ability in tumor cell proliferation, and showed the effect that inhibited the expression of E6 and E7 protein, and the effect that increased the expression of TP53 protein. (Examples 3 to 5). In addition, it was found that the transformants showed excellent serum stability (Example 6).

In another example of the present invention, the effect of the modified siRNAs as a sensitizing agent on radiotherapy was evaluated. That is, siRNAs having the nucleic acid sequences of SEQ ID NOs: 1 to 10 and radiation therapy were co-administered to cervical cancer cell lines. As a result, it showed superior inhibitory effect on tumor cell proliferation, inducing effect on TP53 protein expression, inhibitory effect on E7 protein expression, inducing effect on apoptosis and promotion effect on cell senescence, compared to administration of radiotherapy alone. Thus, it was confirmed that the modified siRNA can exert excellent effects as a sensitizing agent for radiation therapy in the treatment of cancer caused by HPV virus infection (Examples 7 to 10).

In the present invention, oligonucleotides having the nucleic acid sequences of SEQ ID NOs: 1 to 10 are treated with combination therapy for radiotherapy to treat the cancer caused by HPV infection resulting to exert an excellent effect as a sensitizing agent capable of maximizing the therapeutic effect of radiation therapy. The combination therapy may be a method of administering the radiation-sensitizing composition of the present invention sequentially or simultaneously with radiation therapy, and "sequentially" means administering the composition after radiation or administering the radiation-sensitizing composition followed by irradiation. Or it may be, but not limited to, administering the radiation-sensitizing composition two times, one day and two days after irradiation.

Accordingly, the present invention provides a sensitizing composition for radiation therapy, which is administered sequentially or simultaneously with radiation therapy.

The present invention also provides a sensitizing composition for treating HPV(human papilloma virus)-induced cancer with concurrent chemoradiation therapy (CCRT), comprising an oligonucleotide having any one of the nucleic acid sequences selected from the group consisting of SEQ ID NOs: 1 to 10 as an active ingredient.

Concurrent chemoradiation therapy (CCRT) is known to be the main treatment for locally advanced cervical cancer by improving overall survival and disease-free survival rates compared with radiation therapy alone. Concurrent chemoradiation therapy (CCRT) has the advantage that the effects of radiation and anticancer drugs are mutually synergistic, shortening the treatment period and reducing the possibility of cross-resistance. Anticancer drugs play a major role as radio-sensitiser, and cisplatin, 5-FU, paclitaxel and the like are mainly used.

According to the present invention, the sensitizing composition enhances the damage sensitivity of cancer cells to radiation and anticancer drugs in the concurrent chemoradiotherapy of cancer caused by the HPV infection, thereby maximizing the therapeutic effect and reducing the side effect and exhibiting the sensitizing effect.

The present invention also provides a radiation-sensitizing composition comprising the siRNA molecules hybridized SEQ ID NOs: 1 and 2, and the siRNA molecules hybridized with SEQ ID NO: 3 and 4.

The present invention also provides a radiation-sensitizing composition comprising the siRNA molecules hybridized SEQ ID NOs: 5 and 6, the siRNA molecules hybridized SEQ ID NOs: 7 and 8, and the siRNA molecules hybridized with SEQ ID NO: 9 and 10.

Hereinafter, the drawings of the present invention will be described.

FIGs. 1 to 9 show the result that the activity and the serum stability of HPV type 16 or type 18 E6 / E7-specific and chemically modified siRNA having the nucleic acid sequences of SEQ ID NOs: 1 to 10 were shown. Specifically,
(a) FIGs. 1 and 2: The Trypan blue detection test was performed to determine the number of living HeLa cells into which the 2'-OMe modified derivative of siRNA 426 was introduced and the number of living SiHa cells into which the 2'-OMe modified derivative of siRNA 497 was introduced. GFP-specific siRNA (control siRNA) was used as a control;
(b) FIGs. 3 and 4: Silencing efficiency of the 2'-OMe modified siRNA derivatives on the changes in E7 expression and TP53 expression was confirmed using western blot. β-actin was used as a loading control;
(C) FIGs. 5 and 6: they showed qRT-PCR results of E6 and CDKN1A mRNA expression.
(D) FIG. 9: Gel electrophoresis analysis showed serum stability of 2'-OMe modified siRNA derivatives. Unmodified siRNA (lane 0) and modified siRNA 426 derivatives or siRNA 497 derivatives were analyzed by electrophoresis on 15% polyacrylamide gel conditions.

FIGs. 10 to 13 show the results of the synergistic effect of combination therapy of E6 / E7-specific and chemically modified siRNA and radiation therapy on cervical cancer cells. That is, HeLa cells were intracellularly transfected with 20 nM of siRNA variant pairs selected from the group consisting of SEQ ID NOs: 1 to 10 or 10 nM of siRNA Pool (SP), and were subjected to gamma irradiation (γ-irradiation). Similarly, SiHa cells were transfected with 20 nM of siRNA variant pairs selected from the group consisting of SEQ ID NOs: 1 to 10 or siRNA Pool (SP) of 7 nM each, and were subjected to gamma irradiation (γ-irradiation). The number of cancer cells was determined by dividing them into siRNA alone (non-IR) or siRNA combined with gamma irradiation. Cells that did not undergo intracellular transfection and cells into which the control siRNA was introduced were used as controls. More specifically
(a) FIG. 10: Traypan blue detection test indicated live cells under the conditions of cells transfected with siRNA pool, siRNA alone, radiation-siRNA combination;
(b) FIG. 11: Western blot analysis showed the expression of TP53 and HPV E7 proteins (M, mock; C, control siRNA; SP, siRNA pool.);
(c) FIG. 12: Annexin-V and PI binding detection tests indicated the percentage of apoptotic cells among cells transfected with the selected siRNA derivatives;
(d) FIG. 13: it showed the effect of HPV E6 / E7-specific siRNA on cell senescence.

The present invention provides a use of an oligonucleotide having any one of the nucleotide sequences selected from the group consisting of SEQ ID NOs: 1 to 10 for preparing a sensitizing agent for treating HPV(human papilloma virus)-induced cancer with radiation therapy.

The present invention provides a sensitizing method for treating HPV(human papilloma virus)-induced cancer with radiation therapy, the method comprising administering an effective amount of a composition, which comprises an oligonucleotide having any one of the nucleic acid sequences selected from the group consisting of SEQ ID NOs: 1 to 10, to a subject in need thereof.

The present invention is to provide a method of for treating HPV(human papilloma virus)-induced cancer with radiation therapy, the method comprising steps (a) administering an effective amount of a composition, which comprises an oligonucleotide having any one of the nucleic acid sequences selected from the group consisting of SEQ ID NOs: 1 to 10, to a subject in need thereof; and (b) performing a radiotherapy on said subject.

The term "effective amount" of the present invention refers broadly to an amount that increases the effect of cancer treatment when administered to a subject, and it may include healing, substantially preventing, or ameliorating the condition of the cancer. The term "subject" may be an animal, preferably a mammal, especially an animal including a human, and may be an animal-derived cell, tissue, organ, or the like. The subject may be a patient requiring treatment.

The term "treatment" of the present invention refers broadly to ameliorating symptoms due to cancer, and may include healing, substantially preventing, or ameliorating the condition of cancer, including, alleviating, curing or preventing one symptom or most of the symptoms arising from cancer, but not limited to.

The term "sensitizing" of the present invention refers to improving the therapeutic effect of radiation or anticancer agent by improving the sensitivity of cancer cells to radiation or anticancer agent when treating cancer with radiation or an anticancer agent. Preferably, the term "sensitizing" refers to enhance the sensitivity of cancer cells induced by HPV infection to radiation and anticancer agents.

### [ADVANTAGEOUS EFFECT]

In the present invention, the oligonucleotides inhibit the expression of E6 and E7, which are oncogenic proteins of HPV virus, and inhibit the proliferation of tumor cells and has an excellent effect as a sensitizing agent capable of maximizing the apoptotic effect by increasing the sensitivities of tumor cells to radiation.

### [BRIEF DESCRIPTION OF DRAWINGS]

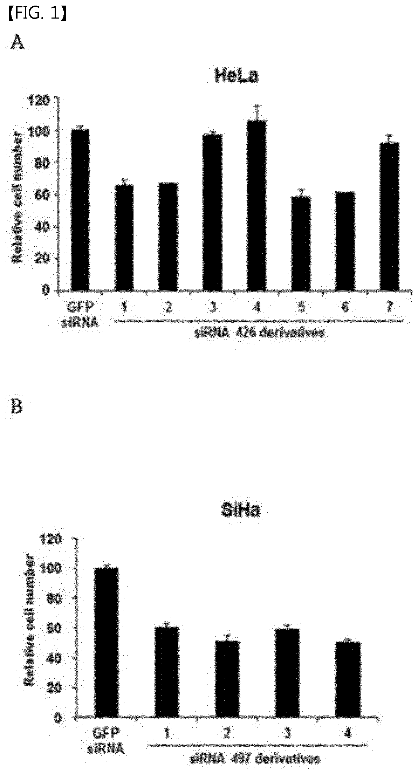

FIG. 1 is a graph showing the inhibitory effect on cell proliferation in a cervical cancer cell line infected with HPV type 18 or type 16 virus by siRNA treatment of the present invention;
(FIG. 1A: Effect on HPV type 18 infected-HeLa cell line, Comparative Example (1, 2, 3, 4, 6 and 7), siRNA 426 hybridized with SEQ ID NOs: 1 and 2 (5)
(FIG. 1B: Effect on HPV type 16 infected-SiHa cell line, Comparative Example (1, 3, and 4), siRNA 497 hybridized with SEQ ID NOs: 9 and 10 (2))

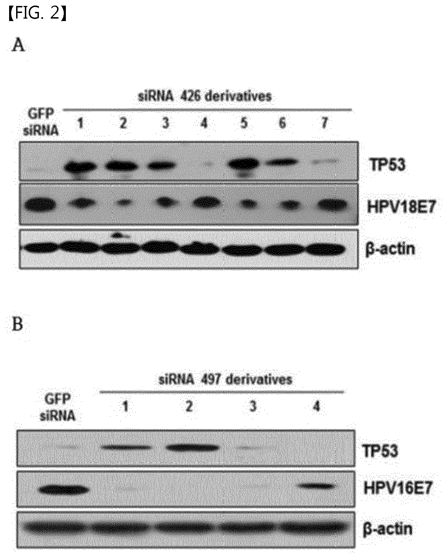

FIG. 2 shows the results of evaluation of expression of TP53 and E7 proteins in cervical cancer cell lines infected with HPV type 18 or type 16 virus by siRNA treatment of the present invention (FIG. 2A: effect on HPV type 18 infected-HeLa cell line, FIG. 2B: effect on HPV type 16 infected-SiHa cell line).

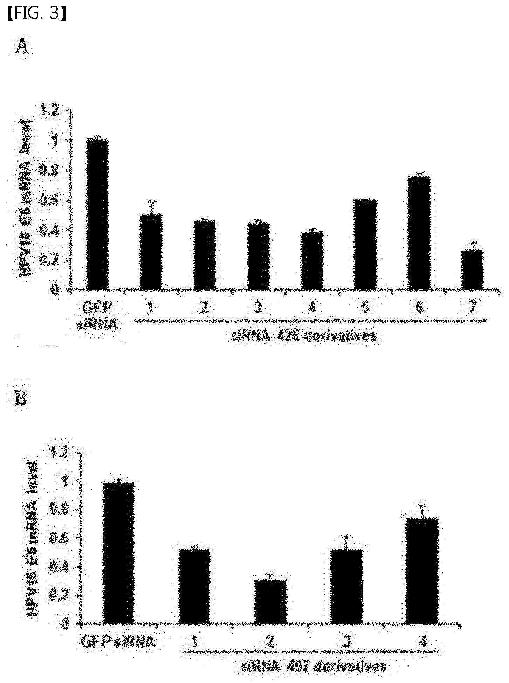

FIG. 3 shows the results of evaluation of expression of E6 mRNA in cervical cancer cell lines infected with HPV type 18 or type 16 virus by siRNA treatment of the present invention (FIG. 3A: effect on HPV 18 infected-HeLa cell line, FIG. 3B: effect on HPV 16 infected-SiHa cell line).

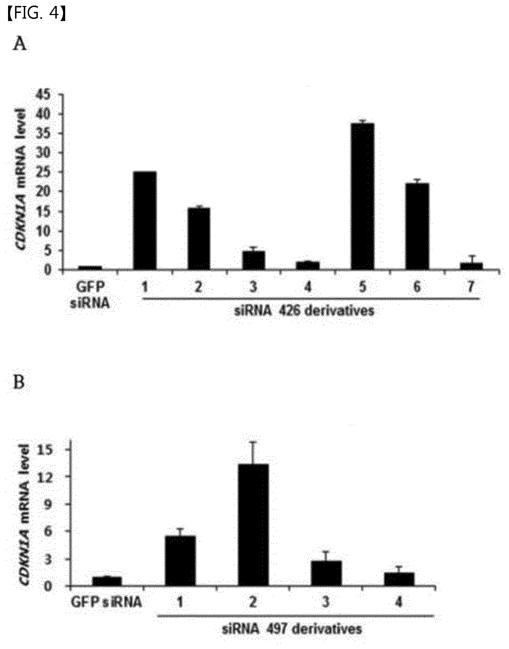

FIG. 4 shows the results of evaluation of expression of CDKN1A mRNA in a cervical cancer cell line infected with HPV type 18 or type 16 virus by siRNA treatment of the present invention (FIG. 4A: effect on HPV 18 infected-HeLa cell line, FIG. 4B: effect on HPV 16 infected-SiHa cell line).

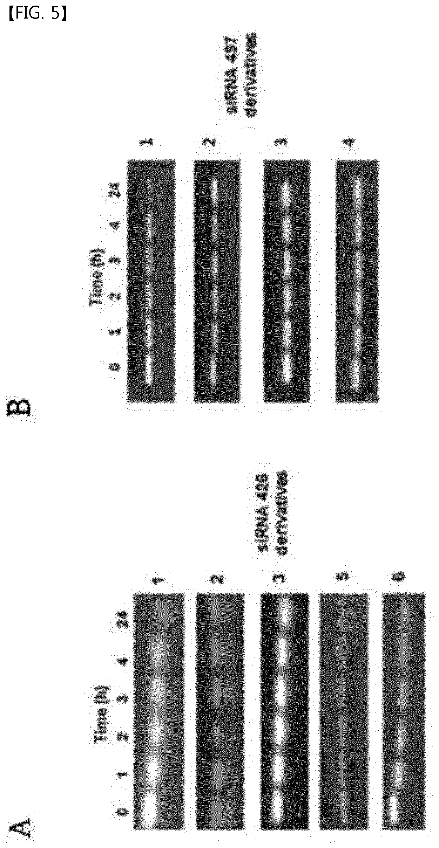

FIG. 5 shows the results of evaluating the plasma stability of the siRNA of the present invention (FIG. 5A: 426 siRNA, FIG. 5B: 497 siRNA).

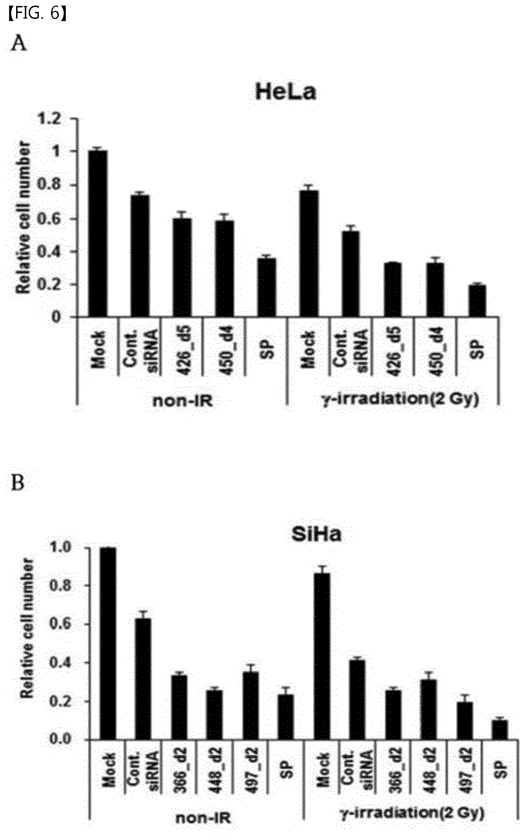

FIG. 6 is a graph showing the inhibitory effect of the combination treatment of siRNA of the present invention and radiation on cell proliferation in a cervical cancer cell line infected with HPV type 18 or type 16 virus (FIG. 6A: effect on HPV type 18 infected-HeLa cell line, FIG. 6B: effect on HPV type 16 infected-SiHa cell line).

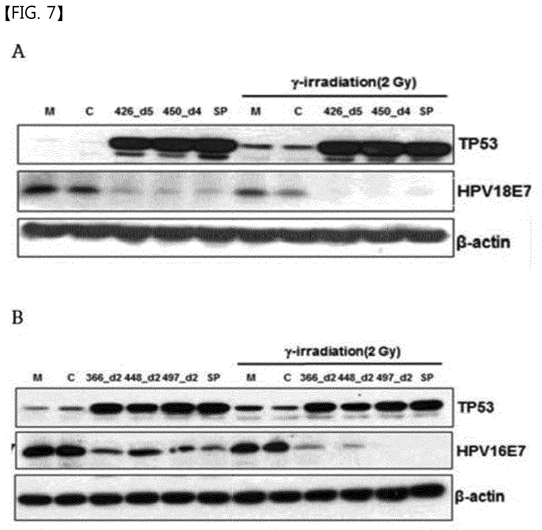

FIG. 7 shows the results of evaluating the expression of TP53 and E7 proteins in cervical cancer cell lines infected with HPV type 18 or type 16 virus by the combination treatment of siRNA of the present invention and radiation (FIG. 7A: effect on HPV type 18 infected-HeLa cell line, Fig. 7B: effect on HPV type 16 infected-SiHa cell line).

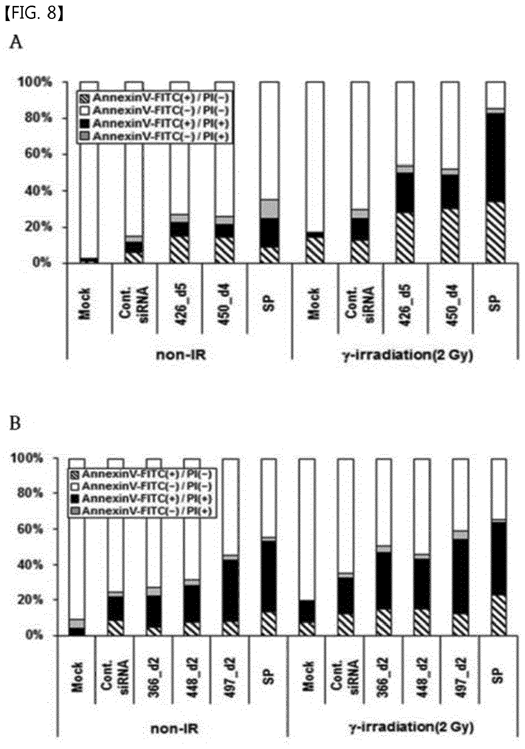

FIG. 8 shows the results of evaluating the apoptosis-inducing effect of cervical cancer cell lines infected with HPV type 18 or type 16 virus by the combination treatment of siRNA of the present invention and radiation (FIG. 8A: effect on HPV type 18 infected-HeLa cell line, Fig. 7B: effect on HPV type 16 infected-SiHa cell line).

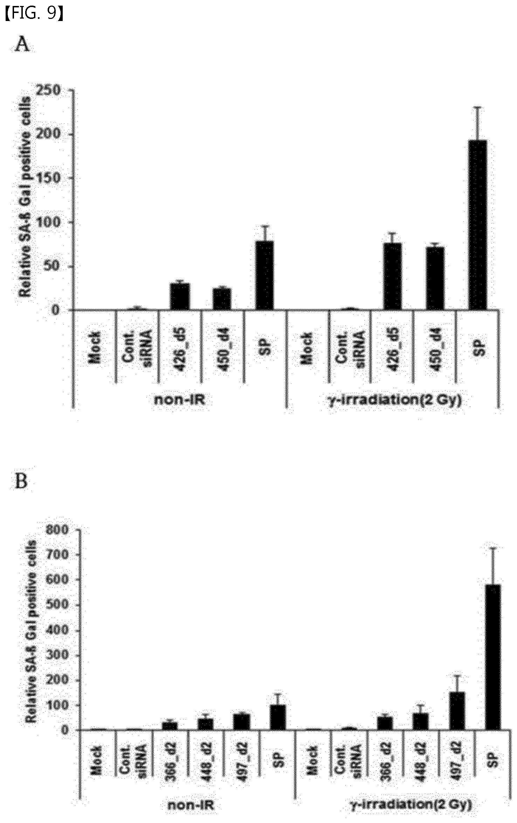

FIG. 9 shows the results of evaluating the effect of inducing cell senescence in cervical cancer cell lines infected with HPV type 18 or type 16 virus by the combination treatment of siRNA of the present invention and radiation (FIG. 9A: effect on HPV type 18 infected-HeLa cell line, Fig. 9B: effect on HPV type 16 infected-SiHa cell line).

### [DETAILED DESCRIPTION OF THE INVENTION]

Hereinafter, the present invention will be described in detail.

However, the following examples are illustrative of the present invention, and the present invention is not limited to the following examples.

### <Experimental method>

### 1. Cell Lines and Western Blotting

HeLa cells are HPV18-positive human cervical cancer cell lines. HeLa-Luc (stably transfected bioluminescent human tumor cell line derived from HeLa cell line) was obtained from Xenogen Corp. (Alameda, CA, USA). The HPV16-positive human cervical cancer cell line, SiHa, was obtained from American Type Culture Collection (Manassas, VA, USA) and validated through short nucleotide sequence repeat. Cells were routinely tested for mycoplasmas.

Whole-cell lysate was extracted using RIPA buffer (10 mM Tris (pH.4), 0.15 M NaCl, 5 mM EDTA, 1% Triton X-100, 0.5% deoxycholic acid sodium salt and 0.1% SDS). After centrifugation, the protein concentration of the supernatant was measured using a BCA protein assay kit (Thermo Fisher Scientific Inc). Protein samples were placed in sample buffer for complete denaturation and boiled for 5 minutes. The samples were then applied to 6%, 10%, or 15% polyacrylamide gels and transferred to a 0.4 µm PVDF membrane (Milipore, Bilecica). After transfer, the membranes were blocked with 5% skim milk, incubated with appropriate concentrations of TP53, E7, β-actin primary antibody and horseradish peroxidase-conjugated IgG. Finally, the expression level of the protein was detected using ECL solution.

### 2. siRNA transfection and γ ray-irradiation

siRNA for HPV18 E6 / E7 (426, 450), HPV16 E6 / E7 (366, 448, 497) and control siRNA were synthesized by BIONEER (Dae-joen, Korea). siRNA transfection was performed with DharmaFect (Dharmacon, Lafayette, CO, USA) and performed according to manufacturer's instructions. Chemically modified (2'-sugar modification by 2'-OMe, 2'-F) siRNA duplex derivatives were designed and synthesized by BIONEER (Dae-jeon, Korea). All siRNAs were diluted with DEPC treated water and the final concentration was 5 ug / uL. For irradiation, the cells were cultured at a density of 5 × 10⁵ cells / 100 mm dish. Twenty-four hours later, cells were plunged into a gamma ray of 2-3 GY from a GC 3000 Elan irradiator (MDS Nordion, Canada). On the next day, the cells were trypsinized and re-plated at a density of 3 x 10⁵ cells / 100 mm dish.

### 3. Flow cytometry analysis and detection of senescence-associated β-galactosidase (SA-β-Gal)

Apoptotic cells were stained with annexin V-fluorescence isothiocyanate (FITC) and Propidium iodide (BD PharMingen, San Diego, Calif., USA) according to manufacturer's instructions and quantitated by flow cytometry. For senesence analysis, the cells were fixed in 2% formaldehyde, 2% glutaraldehyde, and cells were stained at pH 6.0 using X-Gal (5-bromo-4-chloro-3-indolyl-β-D-galactopyranoside) for senesence analysis. The SA-β-Gal positive cells immediately underwent cell count in three representative fields.

### 4. Quantitative real-time PCR analysis

Total RNA was extracted from cells by Trizol RNA isolation method (Invitrogen, Carlsbad, Calif., USA) and then reverse transcribed using oligo-dT primer. Primers and probes for PCR were prepared in TIB MOLBIOL (Berlin, Germany). qRT-PCR was performed using a LightCycler Real-time PCR detection system (Roche Diagnostics, Basel, Switzerland). The expression levels of mRNA in cells and tissues were measured at 530 nm and 705 nm, respectively. The PCR conditions were as follows: 95 °C for 10 minutes, 95 °C for 10 seconds, 45 cycles repeated, 55 °C for 30 seconds, and finally 40 °C for 30 seconds.

### 5. Serum stability

The siRNA double strand (9 ug) was maintained at 90 uL of 10% human serum at 37 °C. 12ul of samples were obtained at each time point (0, 6, 8, 3, 4, 24h), respectively, and frozen and stored at -70 °C immediately. RNA was isolated using unmodified 15% polyacrylamide-TBE, followed by EtBr staining and imaging by UV-transilluminator.

### < Example 1>

### Production of E6 / E7 siRNA of HPV types 16 and 18

The inventors of the present invention prepared siRNAs for E6 / E7 oncogenic proteins of HPV types 16 and 18 as shown in the following Table. In the following Table, the siRNA sequence indicated by 'm' represents a base substituted with a 2'-O-Me modified nucleotide in which a methyl group is bound to a base residue. That is, in case of 2'-0-Me modified U, it is indicated as 'mU', or in case of 2'-O-Me modified G. it is indicated as 'mG'

**[Table 1] siRNA for HPV type16 and 18**

| name | SEQ ID NO: | sequence |
|---|---|---|
| HPV type 18 siRNA 426 | SEQ ID NO: 1 | 5'-CAACCmGAmGCACmGACAmGmGAA-3' |
| | SEQ ID NO: 2 | 5'-UUCCUGUCGUGCUCGGUUG-3' |
| HPV type 18 siRNA 450 | SEQ ID NO: 3 | 5'-CCAACmGACmGCAmGAmGAAACA-3' |
| | SEQ ID NO: 4 | 5'-UGUmUUCUCmUGCGmUCGmUUGG-3' |
| HPV type 16 siRNA 366 | SEQ ID NO: 5 | 5'-GCAAAGACAUCmUmGmGACAAA-3' |
| | SEQ ID NO: 6 | 5'-UUUGUCCAGAUGUCUUUGC-3' |
| HPV type 16 siRNA 448 | SEQ ID NO: 7 | 5'-UCAAmGAACACmGUAmGAmGAAA-3' |
| | SEQ ID NO: 8 | 5'-UUUCUCUACGUGUUCUUGA-3' |
| HPV type 16 siRNA 497 | SEQ ID NO: 9 | 5'-GACCGGUCGAUGUAUGUCUUG-3' |
| | SEQ ID NO: 10 | 5'-AGACAmUACAmUCGACCGGmUCCA-3' |

### <Comparative Example>

### Production of E6 / E7 siRNA of HPV types 16 and 18

The inventors of the present invention compared HPV type 18 siRNA 426 and HPV type 16 siRNA 497 prepared in Example 1 with siRNAs chemically modified in different bases as shown in Table 2 below. In the following Table, an siRNA sequence indicated by 'm' represents a base substituted with a 2'-O-Me modified nucleotide in which a methyl group is bound to a base residue, and the siRNA sequence designated 'f' refers to a 2'-fluorinated base. That is, in the case of 2'-O-Me modified U, it is indicated as 'mU'. In the case of 2'-O-Me modified G, it is indicated as 'mG' or in the case of 2'-fluorinated C, it is indicated as 'fC'.

**[Table 2] siRNA variants for HPV type16 and 18**

| name | SEQ ID NO: | Sequence |
|---|---|---|
| HPV 18 type siRNA 426 | SEQ ID NO: 11 | 5'-CAACCGAGCACGACAGGAA-3' |
| | SEQ ID NO: 12 | 5'-UUCCUGUCGUGCUCGGUUG-3' |
| | SEQ ID NO: 13 | 5'-fCAAfCfCGAGfCAfCGAfCAGGAA-3' |
| | SEQ ID NO: 14 | 5'-UUCCUGUCGUGCUCGGUUG-3' |
| | SEQ ID NO: 15 | 5'-fCAAfCfCGAGfCAfCGAfCAGGAA-3' |
| | SEQ ID NO: 16 | 5'-UUCCmUGmUCGmUGCmUCGGUUG-3' |
| | SEQ ID NO: 17 | 5'-fCAAfCfCGAGfCAfCGAfCAGGAA-3' |
| | SEQ ID NO: 18 | 5'-mUmUCCmUGmUCGmUGCmUCGGUUG-3' |
| | SEQ ID NO: 19 | 5'-CAACCmGAmGCACmGACAmGmGAA-3' |
| | SEQ ID NO: 20 | 5'-UUCCmUGmUCGmUGCmUCGGUUG-3' |
| | SEQ ID NO: 21 | 5'-CAACCmGAmGCACmGACAmGmGAA-3' |
| | SEQ ID NO: 22 | 5'-mUmUCCmUGmUCGmUGCmUCGGUUG-3' |
| HPV 16 type siRNA 497 | SEQ ID NO: 23 | 5'-GACCGGUCGAUGUGUGUCUUG-3' |
| | SEQ ID NO: 24 | 5'-AGACAUACAUCGACCGGUCCA-3' |
| | SEQ ID NO: 25 | 5'-GACCGGmUCGAmUGmUAmUGmUCmUUG-3' |
| | SEQ ID NO: 26 | 5'-AGACAUACAUCGACCGGUCCA-3' |
| | SEQ ID NO: 27 | 5'-GACCGGmUCGAmUGmUAmUGmUCmUUG-3' |
| | SEQ ID NO: 28 | 5'-AGACAmUACAmUCGACCGGmUCCA-3' |

### < Example 2>

### Cell culture and siRNA transfection

Cervical cancer cells, Hela cervical cancer cell lines infected with HPV type 18 virus (HeLa; ATCC CCL-2), SiHa Cervical cancer cell lines infected with HPV type 16 virus (SiHa; ATCC HTB-35) were seeded in 6-well plates at 5 × 10⁴ cells or 1 x 10⁵ cells and cultured in RPMI 1640 or DMEM medium for 24 hours at 37 ° C and 5% carbon dioxide, respectively. siRNA transfection was performed using DarmaFect (Darmacon, Lafayette, CO, USA) and performed according to manufacturer's instructions.

### < Example 3>

### Evaluation of inhibitory effect of siRNA on cell proliferation

siRNA targeting HPV type 16 or E6 / E7 of Example 1 was transfected into cervical cancer cell line infected with the HPV type 18 virus-infected HeLa or HPV type 16 -infected SiHa by the method of Example 2, and after an additional one day of culture, siRNA was transfected again by the method of Example 2. After the transfection, the cells were cultured for 3 days and then the number of cells was measured. GFP siRNA was used as a control.

The results are shown in FIG. 1.

As shown in FIG. 1A, in a HeLa cell line infected with HPV type 18 virus, siRNA 426 hybridized with SEQ ID NOs: 1 and 2 showed an excellent inhibitory effect on cell proliferation (Fig. 1A, No. 5). This effect was confirmed as the best one by comparing with these of siRNA hybridized with SEQ ID NOs: 11 and 12 (No. 1 in FIG. 1A), siRNA hybridized with SEQ ID NOs: 13 and 14 (No. 2 in FIG. 1A), siRNA hybridized with SEQ ID NOs: 15 and 16 (No. 3 in FIG. 1A), siRNA hybridized with SEQ ID NOs: 17 and 18 (No. 4 in FIG. 1A), siRNA hybridized with SEQ ID NOS 19 and 20 (No. 6 in FIG. 1A) and siRNA hybridized with SEQ ID NOS 21 and 22 (No. 7 in FIG. 1A).

As shown in FIG. 1B, in a SiHa cell line infected with HPV type 16 virus, siRNA 497 hybridized with SEQ ID NOs: 9 and 10 showed an excellent inhibitory effect on cell proliferation (No. 2 in FIG. 1B). This effect was confirmed as the best one by comparing with these of siRNA hybridized with SEQ ID NOs: 23 and 24 (No. 1 in FIG. 1B), SEQ ID NOs: 25 and 26 (No. 3 in FIG. 1B), and siRNA hybridized with SEQ ID NOs: 27 and 28 (No. 4 in FIG. 1B).

### < Example 4>

### The effect on TP53 and E7 protein expression

E6 and E7 oncogenic proteins encoded by HPV play an important role in the development of cervical cancer. Most of HPV-associated cervical cancers, unlike other cancers, have a wild-type TP53 gene and levels of TP53 protein in cervical cancers remain significantly low. Because it is consistently degraded by the E6 virus protein as a target. Thus, the expression of TP53 and E7 protein in HPV type 18 or 16 infected cervical cancer cell lines treated with E6 / E7 siRNA of the present invention was confirmed by Western blotting.

The results are shown in FIG. 2.

As shown in FIG. 2, siRNA 426 hybridized with SEQ ID NOs: 1 and 2 in a Hela cell line infected with HPV type 18 virus, and siRNA 497 hybridized with SEQ ID NOs: 9 and 10 in a SiHa cell line infected with HPV type 16 virus, induced strongly the expression of TP53 protein and inhibited the expression of E7 protein. This effect was remarkably superior to the siRNA prepared in Comparative Example.

### < Example 5>

### The effect on E6 and CDKN1A mRNA Expression

The effect of siRNA according to the present invention on mRNA expression of E6 and CDKN1A in HPV-infected cervical cancer cell line was confirmed. The expression of CDKN1A is regulated by the p53 protein, a tumor suppressor gene, and CDKN1A is closely related to the p53-dependent G1 phase cell cycle arrest of the cells. The expression levels of E6 and CDKN1A mRNA were analyzed using TaqMan quantitative real-time PCR (qRT-PCR).

The results are shown in FIGs. 3 and 4.

As shown in FIGs. 3 and 4, siRNA 426 hybridized with SEQ ID NOs: 1 and 2 in a Hela cell line infected with HPV type 18 virus, and siRNA 497 hybridized with SEQ ID NOs: 9 and 10 in a SiHa cell line infected with HPV type 16 virus, inhibited most effectively the expression of E6 mRNA and induced most effectively the expression of CDKN1A mRNA.

### <Example 6>

### Serum stability evaluation of siRNA

As shown in FIG. 5, the serum stability of siRNA 426 hybridized with SEQ ID NOs: 1 and 2 and siRNA 497 hybridized with SEQ ID NOs: 9 and 10 were found to be superior to that of siRNA that was not chemically modified (SiRNA hybridized with SEQ ID NOs: 11 and 12 in FIG. 5A, No. 1 and siRNA hybridized with SEQ ID NOs: 23 and 24) in FIG. 5B, No. 1.

### <Example 7>

### The inhibitory effects of combination treatment with siRNA and radiation on cell proliferation

Experiments were conducted to confirm whether siRNAs having the nucleic acid sequences of SEQ ID NOs: 1 to 10 exhibited the effect as a sensitizing agent for combination therapy with radiation. siRNAs having the nucleic acid sequences of SEQ ID NOs: 1 to 10 were transfected into HeLa cells or SiHa cells, respectively, in the same manner as in Example 3, and then radiation was applied to evaluate the effects of combination therapy.

The results are shown in FIG. 6.

As shown in FIG. 6A, in HeLa cells infected with HPV type 18 virus, the group of combination therapy of siRNA 426 (426_d5 in FIG. 6A) or siRNA 450 (450_d4 in FIG. 6A) and irradiation showed more than two-fold improved inhibitory effect on cell proliferation when compared to the group treated with siRNA 426 or siRNA 450 alone. In addition, the group of combination therapy of siRNA 426 or siRNA 450 and irradiation showed more than two-fold improved inhibitory effect on cell proliferation when compared to the mock control group that was irradiated only. Therefore, it was confirmed that siRNA 426 or siRNA 450 showed excellent sensitizing effect on radiotherapy.

As shown in FIG. 6B, in SiHa cells infected with HPV type 16 virus, the group of combination therapy of siRNA 366 (366_d2 in FIG. 6B), siRNA 448 (448_d2 in FIG. 6B) and siRNA 497 (497_d2 in FIG. 6B) and irradiation showed more than two-fold improved inhibitory effect on cell proliferation when compared to the mock control group that was irradiated only. Therefore, it was confirmed that siRNA 366, siRNA 448 and siRNA 497 exhibited excellent sensitizing effect on radiotherapy.

### <Example 8>

### Changes of TP53 and E7 Protein Expression by Combination Treatment of siRNA and Radiation

The expression of TP53 and E7 protein was evaluated by the same method as in Example 4, by combination treatment of siRNA and radiation.

The results are shown in FIG. 7.

As shown in FIG. 7, the expression of TP53 protein was significantly increased and the expression of E7 protein was significantly decreased in the group co-treated with siRNA 426, siRNA 450 siRNA 366, siRNA 448 or siRNA 497 and radiation, therefore, it was confirmed that siRNA 426, siRNA 450 siRNA 366, siRNA 448 and siRNA 497 exhibited excellent sensitizing effect on radiotherapy.

### <Example 9>

### Inducing effect on cell apoptosis by combination treatment of siRNA and radiation

In the same method as described above, siRNA was transfected alone into HeLa cells or SiHa cell lines, or combined with radiotherapy, and cultured for one day. Cells were stained with Annexin V and PI (propidium iodide) reagents using a cell death kit (BD, USA), and reacted at room temperature for 30 minutes. Cell death was confirmed using a flow cytometer.

The results are shown in FIG. 8.

As shown in FIG. 8, annexin-v-positive apoptotic cells were significantly increased in the group of combination therapy of siRNA 426, siRNA 450 siRNA 366, siRNA 448 or siRNA 497 and irradiation when compared to the mock control group that was irradiated only. Therefore, it was confirmed that siRNAs of the present invention were effective as a sensitizing agent for increasing the apoptotic effect of radiation therapy on cancer cells.

### <Example 10>

### Inducing effect on cell senescence by combination treatment of siRNA and radiation

In the same method as described above, siRNA was transfected alone into HeLa cells or SiHa cell lines, or combined with radiotherapy, and cultured for one day. For senescence analysis, cells were fixed in 2% formaldehyde / 0.2% glutaraldehyde, and cells were stained at pH 6.0 using X-Gal (5-bromo-4-chloro-3-indolyl-β-D-galactopyranoside) as previously performed in the method. The SA-β-Gal positive cells immediately underwent cell count in three representative fields.

The results are shown in FIG. 9.

As shown in FIG. 9, the group of combination therapy of siRNA 426, siRNA 450 siRNA 366, siRNA 448 or siRNA 497 and irradiation showed SA-β Gal activity increased by 10-20 folds when compared to the mock control group that was irradiated only. Therefore, it was confirmed that the siRNA variants of the present invention showed excellent sensitizing effect on radiotherapy.

### <Example 11>

### Evaluation of radiation sensitization effect of siRNA pool (SP)

In nature, siRNAs generated by Dicer represent a siRNA pool that silence together the expression of the gene. The siRNA pool obtained in this way is an efficient silencer and has a low off-target effect. The inventors of the present invention evaluated the apoptotic effects of HPV type 18 siRNA pool (SP) using 10 nM of siRNA described above and 7 nM and 10 nM of HPV type 16 siRNA pool (SP), respectively.

As shown in FIGs. 6 to 9, the siRNA pool (SP) showed excellent inhibitory effect of cell proliferation, induction effect of TP53 protein expression, inhibitory effect of E7 protein expression, induction effect of apoptosis, and induction effect of cell senescence, and it was confirmed that these effects were maximized by radiation therapy.

Therefore, it is considered that the siRNA pool in which each of the siRNA variants according to the present invention is mixed can exhibit an excellent radiation sensitizatizing effect while exhibiting low side effects.

### [INDUSTRIAL AVAILABILITY]

The oligonucleotides of the present invention inhibit the expression of E6 and E7, which are oncogenic proteins of HPV virus, resulting in inhibiting the proliferation of tumor cells and enhancing the sensitivity of tumor cells to radiation, thus, they have excellent effect as a sensitizing agent capable of maximizing the apoptosis effect and are highly applicable to be used in industry.

## Claims

1. A sensitizing composition for treating HPV(human papilloma virus)-induced cancer with radiation therapy, comprising an oligonucleotide having any one of the nucleic acid sequences selected from the group consisting of SEQ ID NOs: 1 to 10 as an active ingredient.

2. The composition according to claim 1, wherein the oligonucleotide is a small interfering RNA (siRNA) complementarily hybridized with a sense oligonucleotide and a complementary antisense oligonucleotide.

3. The composition according to claim 1, wherein HPV(human papilloma virus)-induced cancer is selected from the group consisting of cervical cancer, vaginal cancer, vulvar cancer, anal cancer, penis cancer, tonsillar cancer, pharynx cancer, laryngeal cancer, head & neck cancer, and adenocarcinoma of lung.

4. The composition of claim 1, wherein the composition is administered sequentially or concurrently with radiation therapy.

5. The composition of claim 1, wherein the composition is for concurrent chemoradiation therapy.

6. The composition of claim 1, wherein said composition comprises all of siRNA molecules hybridized with SEQ ID NOs: 1 and 2, and siRNA molecules hybridized with SEQ ID NOs: 3 and 4.

7. The composition of claim 1, wherein said composition comprises all of siRNA molecules hybridized with SEQ ID NOs: 5 and 6, siRNA molecules hybridized with SEQ ID NOs: 7 and 8, and siRNA molecules hybridized with SEQ ID NOs: 9 and 10.

8. A sensitizing composition for treating HPV(human papilloma virus)-induced cancer with radiation therapy, comprising an oligonucleotide having any one of the nucleic acid sequences selected from the group consisting of SEQ ID NOs: 1 to 10 as an active ingredient.

9. Use of an oligonucleotide having any one of the nucleotide sequences selected from the group consisting of SEQ ID NOs: 1 to 10 for preparing a sensitizing agent for treating HPV(human papilloma virus)-induced cancer with radiation therapy.

10. A sensitizing method for treating HPV(human papilloma virus)-induced cancer with radiation therapy, the method comprising administering to a subject in need thereof an effective amount of a composition which comprises an oligonucleotide having any one of the nucleic acid sequences selected from the group consisting of SEQ ID NOs: 1 to 10.

11. The method according to claim 10, wherein the oligonucleotide is a small interfering RNA (siRNA) complementarily hybridized with a sense oligonucleotide and a complementary antisense oligonucleotide.

12. The method according to claim 10, wherein HPV(human papilloma virus)-induced cancer is selected from the group consisting of cervical cancer, vaginal cancer, vulvar cancer, anal cancer, penis cancer, tonsillar cancer, pharynx cancer, laryngeal cancer, head & neck cancer, and adenocarcinoma of lung.

13. The method of claim 10, wherein the composition is administered sequentially or concurrently with radiation therapy.

14. The method of claim 10, wherein the composition is for concurrent chemoradiation therapy.

15. A method of for treating HPV(human papilloma virus)-induced cancer with radiation therapy, the method comprising steps (a) administering to a subject in need thereof an effective amount of a composition which comprises an oligonucleotide having any one of the nucleic acid sequences selected from the group consisting of SEQ ID NOs: 1 to 10 ; and (b) performing a radiotherapy on said subject.

16. The method according to claim 15, wherein HPV(human papilloma virus)-induced cancer is selected from the group consisting of cervical cancer, vaginal cancer, vulvar cancer, anal cancer, penis cancer, tonsillar cancer, pharynx cancer, laryngeal cancer, head & neck cancer, and adenocarcinoma of lung.

17. The method of claim 15, wherein the radiation therapy is concurrent chemoradiation therapy.


<110>    ABION Inc.

<120>    COMPOSITION FOR CANCER CELL SENSITIZATION CONTAINING AS ACTIVE
         INGREDIENT SUBSTANCE INHIBITING EXPRESSION OF ONCOGENE OF HPV
         VIRUS

<130>    P45480-WOEP

<140>    EP16792957.9
<141>    2016-05-10

<150>    10-2015-0066218
<151>    2015-05-12

<160>    28

<170>    KopatentIn 2.0

<210>    1
<211>    19
<212>    RNA
<213>    Artificial Sequence

<220>
<223>    synthetic oligonucleotide


<220>
<221>    misc_feature
<222>    (6)
<223>    n denotes o methyl modified guanine


<220>
<221>    misc_feature
<222>    (8)
<223>    n denotes o methyl modified guanine


<220>
<221>    misc_feature
<222>    (12)
<223>    n denotes o methyl modified guanine


<220>
<221>    misc_feature
<222>    (16)
<223>    n denotes o methyl modified guanine


<220>
<221>    misc_feature
<222>    (17)
<223>    n denotes o methyl modified guanine


<400>    1
caaccnanca cnacannaa                                                    19


<210>    2
<211>    19

<212>    RNA
<213>    Artificial Sequence

<220>
<223>    HPV type 18 siRNA 426 reverse


<400>    2
uuccugucgu gcucgguug                                                    19


<210>    3
<211>    19
<212>    RNA
<213>    Artificial Sequence

<220>
<223>    HPV type 18 siRNA 450 forward


<220>
<221>    misc_feature
<222>    (6)
<223>    n denotes o methyl modified guanine


<220>
<221>    misc_feature
<222>    (9)
<223>    n denotes o methyl modified guanine


<220>
<221>    misc_feature
<222>    (12)
<223>    n denotes o methyl modified guanine


<220>
<221>    misc_feature
<222>    (14)
<223>    n denotes o methyl modified guanine


<400>    3
ccaacnacnc ananaaaca                                                    19


<210>    4
<211>    19
<212>    RNA
<213>    Artificial Sequence

<220>
<223>    HPV type 18 siRNA 450 reverse


<220>
<221>    misc_feature
<222>    (4)
<223>    n denotes o methyl modified uracil

<220>
<221>    misc_feature
<222>    (9)
<223>    n denotes o methyl modified uracil


<220>
<221>    misc_feature
<222>    (13)
<223>    n denotes o methyl modified uracil


<220>
<221>    misc_feature
<222>    (16)
<223>    n denotes o methyl modified uracil


<400>    4
ugunucucng cgncgnugg                                                    19


<210>    5
<211>    19
<212>    RNA
<213>    Artificial Sequence

<220>
<223>    HPV type 16 siRNA 366 forward


<220>
<221>    misc_feature
<222>    (12)
<223>    n denotes o methyl modified uracil


<220>
<221>    misc_feature
<222>    (13)..(14)
<223>    n denotes o methyl modified guanine


<400>    5
gcaaagacau cnnnacaaa                                                    19


<210>    6
<211>    19
<212>    RNA
<213>    Artificial Sequence

<220>
<223>    HPV type 16 siRNA 366 reverse


<400>    6
uuuguccaga ugucuuugc                                                    19


<210>    7
<211>    19
<212>    RNA

<213>    Artificial Sequence

<220>
<223>    HPV type 16 siRNA 448 forward


<220>
<221>    misc_feature
<222>    (5)
<223>    n denotes o methyl modified guanine


<220>
<221>    misc_feature
<222>    (11)
<223>    n denotes o methyl modified guanine


<220>
<221>    misc_feature
<222>    (14)
<223>    n denotes o methyl modified guanine


<220>
<221>    misc_feature
<222>    (16)
<223>    n denotes o methyl modified guanine


<400>    7
ucaanaacac nuananaaa                                                    19


<210>    8
<211>    19
<212>    RNA
<213>    Artificial Sequence

<220>
<223>    HPV type 16 siRNA 448 reverse


<400>    8
uuucucuacg uguucuuga                                                    19


<210>    9
<211>    21
<212>    RNA
<213>    Artificial Sequence

<220>
<223>    HPV type 16 siRNA 497 forward


<400>    9
gaccggucga uguaugucuu g                                                 21


<210>    10
<211>    21
<212>    RNA

<213>    Artificial Sequence

<220>
<223>    HPV type 16 siRNA 497 reverse


<220>
<221>    misc_feature
<222>    (6)
<223>    n denotes o methyl modified uracil


<220>
<221>    misc_feature
<222>    (10)
<223>    n denotes o methyl modified uracil


<220>
<221>    misc_feature
<222>    (18)
<223>    n denotes o methyl modified uracil


<400>    10
agacanacan cgaccggncc a                                                  21


<210>    11
<211>    19
<212>    RNA
<213>    Artificial Sequence

<220>
<223>    synthetic oligonucleotide


<400>    11
caaccgagca cgacaggaa                                                     19


<210>    12
<211>    19
<212>    RNA
<213>    Artificial Sequence

<220>
<223>    synthetic oligonucleotide


<400>    12
uuccugucgu gcucgguug                                                     19


<210>    13
<211>    19
<212>    RNA
<213>    Artificial Sequence

<220>
<223>    synthetic oligonucleotide

<220>
<221>    misc_feature
<222>    (1)
<223>    n denotes F modified cytosine


<220>
<221>    misc_feature
<222>    (4)
<223>    n denotes F modified cytosine


<220>
<221>    misc_feature
<222>    (5)
<223>    n denotes F modified cytosine


<220>
<221>    misc_feature
<222>    (9)
<223>    n denotes F modified cytosine


<220>
<221>    misc_feature
<222>    (11)
<223>    n denotes F modified cytosine


<220>
<221>    misc_feature
<222>    (14)
<223>    n denotes F modified cytosine


<400>    13
naanngagna nganaggaa                                                    19


<210>    14
<211>    19
<212>    RNA
<213>    Artificial Sequence

<220>
<223>    synthetic oligonucleotide


<400>    14
uuccugucgu gcucgguug                                                    19


<210>    15
<211>    19
<212>    RNA
<213>    Artificial Sequence

<220>
<223>    synthetic oligonucleotide


<220>

<221>    misc_feature
<222>    (1)
<223>    n denotes F modified cytosine

<220>
<221>    misc_feature
<222>    (4)..(5)
<223>    n denotes F modified cytosine

<220>
<221>    misc_feature
<222>    (9)
<223>    n denotes F modified cytosine

<220>
<221>    misc_feature
<222>    (11)
<223>    n denotes F modified cytosine

<220>
<221>    misc_feature
<222>    (14)
<223>    n denotes F modified cytosine

<400>    15
naanngagna nganaggaa                                                    19

<210>    16
<211>    19
<212>    RNA
<213>    Artificial Sequence

<220>
<223>    synthetic oligonucleotide

<220>
<221>    misc_feature
<222>    (5)
<223>    n denotes o methyl modified uracil

<220>
<221>    misc_feature
<222>    (7)
<223>    n denotes o methyl modified uracil

<220>
<221>    misc_feature
<222>    (10)
<223>    n denotes o methyl modified uracil

<220>
<221>    misc_feature
<222>    (13)

<223>    n denotes o methyl modified uracil

<400>    16
uuccngncgn gcncgguug                                                    19

<210>    17
<211>    19
<212>    RNA
<213>    Artificial Sequence

<220>
<223>    synthetic oligonucleotide

<220>
<221>    misc_feature
<222>    (1)
<223>    n denotes F modified cytosine

<220>
<221>    misc_feature
<222>    (4)..(5)
<223>    n denotes F modified cytosine

<220>
<221>    misc_feature
<222>    (9)
<223>    n denotes F modified cytosine

<220>
<221>    misc_feature
<222>    (11)
<223>    n denotes F modified cytosine

<220>
<221>    misc_feature
<222>    (14)
<223>    n denotes F modified cytosine

<400>    17
naanngagna nganaggaa                                                    19

<210>    18
<211>    19
<212>    RNA
<213>    Artificial Sequence

<220>
<223>    synthetic oligonucleotide

<220>
<221>    misc_feature
<222>    (1)..(2)
<223>    n denotes o methyl modified uracil

<220>
<221>    misc_feature
<222>    (5)
<223>    n denotes o methyl modified uracil


<220>
<221>    misc_feature
<222>    (7)
<223>    n denotes o methyl modified uracil


<220>
<221>    misc_feature
<222>    (10)
<223>    n denotes o methyl modified uracil


<220>
<221>    misc_feature
<222>    (13)
<223>    n denotes o methyl modified uracil


<400>    18
nnccngncgn gcncgguug                                                    19


<210>    19
<211>    19
<212>    RNA
<213>    Artificial Sequence

<220>
<223>    synthetic oligonucleotide


<220>
<221>    misc_feature
<222>    (6)
<223>    n denotes o methyl modified guanine


<220>
<221>    misc_feature
<222>    (8)
<223>    n denotes o methyl modified guanine


<220>
<221>    misc_feature
<222>    (12)
<223>    n denotes o methyl modified guanine


<220>
<221>    misc_feature
<222>    (16)
<223>    n denotes o methyl modified guanine

<220>
<221>    misc_feature
<222>    (17)
<223>    n denotes o methyl modified guanine


<400>    19
caaccnanca cnacannaa                                                    19


<210>    20
<211>    19
<212>    RNA
<213>    Artificial Sequence

<220>
<223>    synthetic oligonucleotide


<220>
<221>    misc_feature
<222>    (5)
<223>    n denotes o methyl modified uracil


<220>
<221>    misc_feature
<222>    (7)
<223>    n denotes o methyl modified uracil


<220>
<221>    misc_feature
<222>    (10)
<223>    n denotes o methyl modified uracil


<220>
<221>    misc_feature
<222>    (13)
<223>    n denotes o methyl modified uracil


<400>    20
uuccngncgn gcncgguug                                                    19


<210>    21
<211>    19
<212>    RNA
<213>    Artificial Sequence

<220>
<223>    synthetic oligonucleotide


<220>
<221>    misc_feature
<222>    (6)
<223>    n denotes o methyl modified guanine


<220>

<221>    misc_feature
<222>    (8)
<223>    n denotes o methyl modified guanine

<220>
<221>    misc_feature
<222>    (12)
<223>    n denotes o methyl modified guanine

<220>
<221>    misc_feature
<222>    (16)..(17)
<223>    n denotes o methyl modified guanine

<400>    21
caaccnanca cnacannaa                                                    19

<210>    22
<211>    19
<212>    RNA
<213>    Artificial Sequence

<220>
<223>    synthetic oligonucleotide

<220>
<221>    misc_feature
<222>    (1)..(2)
<223>    n denotes o methyl modified uracil

<220>
<221>    misc_feature
<222>    (5)
<223>    n denotes o methyl modified uracil

<220>
<221>    misc_feature
<222>    (7)
<223>    n denotes o methyl modified uracil

<220>
<221>    misc_feature
<222>    (10)
<223>    n denotes o methyl modified uracil

<220>
<221>    misc_feature
<222>    (13)
<223>    n denotes o methyl modified uracil

<400>    22
nnccngncgn gcncgguug                                                    19

<210>    23
<211>    21
<212>    RNA
<213>    Artificial Sequence

<220>
<223>    synthetic oligonucleotide


<400>    23
gaccggucga ugugugucuu g                                                  21


<210>    24
<211>    21
<212>    RNA
<213>    Artificial Sequence

<220>
<223>    synthetic oligonucleotide


<400>    24
agacauacau cgaccggucc a                                                  21


<210>    25
<211>    21
<212>    RNA
<213>    Artificial Sequence

<220>
<223>    synthetic oligonucleotide


<220>
<221>    misc_feature
<222>    (7)
<223>    n denotes o methyl modified uracil


<220>
<221>    misc_feature
<222>    (11)
<223>    n denotes o methyl modified uracil


<220>
<221>    misc_feature
<222>    (13)
<223>    n denotes o methyl modified uracil


<220>
<221>    misc_feature
<222>    (15)
<223>    n denotes o methyl modified uracil


<220>
<221>    misc_feature
<222>    (17)

<223>    n denotes o methyl modified uracil

<220>
<221>    misc_feature
<222>    (19)
<223>    n denotes o methyl modified uracil

<400>    25
gaccggncga ngnangncnu g                                                21

<210>    26
<211>    21
<212>    RNA
<213>    Artificial Sequence

<220>
<223>    synthetic oligonucleotide

<400>    26
agacauacau cgaccggucc a                                                21

<210>    27
<211>    21
<212>    RNA
<213>    Artificial Sequence

<220>
<223>    synthetic oligonucleotide

<220>
<221>    misc_feature
<222>    (7)
<223>    n denotes o methyl modified uracil

<220>
<221>    misc_feature
<222>    (11)
<223>    n denotes o methyl modified uracil

<220>
<221>    misc_feature
<222>    (13)
<223>    n denotes o methyl modified uracil

<220>
<221>    misc_feature
<222>    (15)
<223>    n denotes o methyl modified uracil

<220>
<221>    misc_feature
<222>    (17)
<223>    n denotes o methyl modified uracil

<220>
<221>    misc_feature
<222>    (19)
<223>    n denotes o methyl modified uracil

<400>    27
gaccggncga ngnangncnu g                                                21

<210>    28
<211>    21
<212>    RNA
<213>    Artificial Sequence

<220>
<223>    synthetic oligonucleotide

<220>
<221>    misc_feature
<222>    (6)
<223>    n denotes o methyl modified uracil

<220>
<221>    misc_feature
<222>    (10)
<223>    n denotes o methyl modified uracil

<220>
<221>    misc_feature
<222>    (18)
<223>    n denotes o methyl modified uracil

<400>    28
agacanacan cgaccggncc a                                                21